# EUROPEAN PATENT APPLICATION

(11) **EP 2 692 343 A1**
(43) Date of publication of application: **05.02.2014**
(21) Application number: 12179232.9
(22) Date of filing: 03.08.2012
(51) Int. Cl.: A61K 31/215, A61K 31/275, A61K 9/00, A61P 29/00

(54) **Combination therapy for treatment of multiple sclerosis**

(71) Applicant: Forward Pharma A/S, 1100 København K (DK)
(72) Inventor: Terwey, Dr. med. Theis, 10115 Berlin (DE); Rupp, Dr. Ronald, 51467 Bergisch Gladbach (DE); Andersen, MD, Peder M., 1561 Copenhagen V (DK)
(74) Representative: HOFFMANN EITLE

(57) **Abstract**

The present invention relates to a method of treating MS in a human patient in need of such treatment and comprises administering to said patient a combination therapy in a single oral dosage form (e.g. a tablet or capsule) of dimethylfumarate and teriflunomide (or its prodrug leflunomide) which is more effective than the single agents alone and/or has reduced side effects and better tolerability than the single agents alone and/or can be given in a reduced frequency. Moreover, the present invention is directed to a pharmaceutical composition suitable for the oral treatment of multiple sclerosis consisting of dimethylfumarate and teriflunomide as active ingredients and one or more pharmaceutically acceptable excipients.

## Description

### FIELD OF THE INVENTION

The invention relates to pharmaceutical compositions for oral use comprising a fixed combination of a first active pharmaceutical ingredient of dimethylfumarate or a pharmaceutically acceptable administration form thereof and a second active pharmaceutical ingredient of teriflunomide or a pharmaceutically acceptable administration form thereof and to the use of such compositions in treating multiple sclerosis. The use of dimethylfumarate in combination with teriflunomide according to this invention allows lowering the dose of one or both agents below levels previously believed to be necessary for efficacy, while achieving better efficacy with comparable adverse effects than seen for the individual agents or achieving similar efficacy with potentially less adverse effects than seen for the individual agents. The combinations according to the present invention may also allow for a reduced dosing frequency.

### BACKGROUND

Multiple sclerosis (MS) is a chronic inflammatory disease that attacks myelinated axons in the central nervous system (CNS). It is thought that MS is caused by a T-cell triggered, autoimmune inflammatory reaction with additional B cell activation, involvement of monocytes and macrophages, secretion of cytokines and breakdown of the blood-brain barrier. When myelin is lost nerves can no longer effectively conduct signals which can lead to a plethora of clinical symptoms including sensory defects, motor dysfunctions, visual impairments, bladder and bowel difficulties, sexual dysfunction, fatigue, and even cognitive impairment.

Initially, most cases of MS follow a relapsing-remitting pattern where short episodes of neurologic exacerbations resolve completely but relapses occur (relapsing-remitting MS, RRMS). Later, approximately half of patients develop a continuously progressive pattern with often permanent disability (secondary progressive MS, SPMS). Some cases of MS follow a continuously progressive pattern without remission phases already from the beginning (primary progressive MS, PPMS). Other cases have periods of acute exacerbations while proceeding along a course of increasing neurological deficits without remissions (progressive-relapsing MS, PRMS). The onset of the disease is usually in young adults and it is more common in women. About 2-2.5 million people are living with MS worldwide.

The treatment of choice for exacerbations is generally high doses of corticosteroids. The treatment of the chronic progression of MS aims to target the underlying immune disorder with the goal to reduce the frequency of relapses, to reduce the progression of disability and to preserve brain structure. The available treatments are generally based on immunosuppressive and immunomodulatory mechanisms while for some drugs additional direct neuroprotective effects are postulated.

Treatment success in clinical trials is primarily measured by the reduction in annual relapse rate (ARR) while other commonly used endpoints include time to disability progression as assessed by the Expanded Disability Status Scale (EDSS) or reduction in new brain lesions as measured by brain magnetic resonance imaging (MRI).

All currently available agents are only approved for the relapsing-remitting form of MS. The first agents were all injectable drugs (FDA approved are Interferon beta-1a (Avonex, Rebif), Interferon beta-1b (Betaseron, Extavia), Glatiramer acetate (Copaxone) and Natalizumab (Tysabri)) and only recently in 2010 an oral drug received an MS label (Fingolimod (Gilenya)). In addition, MS is treated with chemotherapeutic agents such as the FDA approved Mitoxantrone (Novantrone) or off-label azathioprine, methotrexate, cladribine and cyclophosphamide.

Besides the approved oral drug fingolimod, various other oral agents are in clinical development, the most advanced being dimethylfumarate and teriflunomide, both having completed Phase III studies.

Dimethylfumarate (trans-1,2-Ethylenedicarboxylic acid dimethyl ester, occasionally also abbreviated as BG-12 or DMF) belongs to the class of fumaric acid esters (FAE) and appears to have the most attractive safety profile and good efficacy based on two randomized, double-blind, placebo-controlled, dose-comparison Phase III studies with overall more than 2600 patients (DEFINE study (R. Gold et al., Mult Scler 2011 Oct; 17(10)S34) and CONFIRM study (Fox et al., Neurology 2012 78:S01.003). Both studies evaluated dimethylfumarate (BG-12) 240 mg twice daily (BID) and three times daily (TID) versus placebo while the CONFIRM study also included an active, reference comparator arm with subcutaneous glatiramer acetate (GA) 20 mg daily.

Regarding efficacy 240 mg BID and 240 mg TID of dimethylfumarate appeared to be superior to the most widely used conventional agents the Interferons (based on indirect comparison) and Glatiramer acetate (based on direct comparison in the CONFIRM trial), but still many patients do experience relapses and progression of disability and may require subsequent therapy with more effective but potentially also more harmful intravenous agents such as Natalizumab (Tysabri, Biogen Idec) or off-label Alemtuzumab (Campath, Sanofi).

Regarding safety the studies found that the incidence of adverse events, serious adverse events, including serious infections, and discontinuations due to adverse effects were similar across all study groups, including placebo. This excellent safety profile is supported by more than 150.000 patient years experience with another DMF-containing drug, Fumaderm, which has been approved for psoriasis in Germany in 1994 (Morwietz et al., J Dtsch Dermatol Ges. 2007 Aug;5(8):716-7). Despite promising long-term safety data dimethylfumarate is associated with some short term tolerability issues, mainly diarrhea and flushing, which can lead to discontinuation of the drug in some patients.

In more detail, CONFIRM, which analyzed the safety and efficacy of dimethylfumarate 120 mg po (per os = oral) capsule BID or TID vs. placebo vs. glatiramer acetate 20 mg sc (subcutaneously) once-daily in 1430 patients with RRMS, showed that dimethylfumarate met the primary endpoint by significantly reducing annualized relapse rate by 44% and 51% for NID and TID, respectively versus placebo. It also met all secondary relapse and MRI endpoints in both dose regimens. Dimethylfumarate BID and TID reduced the number of new or newly enlarging T2-hyperintense lesions by 71 and 73%, new T1-hypointense lesions by 57 and 65% and the proportion of patients who relapsed by 34 and 45% compared to 54, 41 and 29% for glatiramer acetate, respectively. Dimethylfumarate also reduced 12-week confirmed disability progression as measured by EDSS by 21% for BID and 24% for TID at 2 years compared to 7% for placebo and glatiramer acetate. The most common adverse effects in the dimethylfumarate groups were flushing and gastrointestinal (GI) events. There were no malignancies in the dimethylfumarate groups. The incidence of these events decreased substantially in the dimethylfumarate groups after the 1st month. The most frequently reported serious adverse effect was MS relapse, with no other events reported by more than 2 patients in any group (Press releases, Biogen, and Fox et al., Neurology 2012 78:501.003).

DEFINE, which analyzed the efficacy and safety of dimethylfumarate 240 po capsules BID and TID in 1237 patients with RRM showed a significant reduction in the proportion of patients with RRMS who relapsed at 2 years compared with placebo (primary endpoint, 49% reduction versus placebo for BID and 50% reduction versus placebo for TID). Both doses of dimethylfumarate showed a significant reduction in annualized relapse rate (53% reduction versus placebo for BID and 48% reduction versus placebo for TID), in the number of new or newly enlarging T2 hyperintense lesions, in new gadolinium-enhancing (Gd+) lesions, and in the rate of disability progression as measured by the Expanded Disability Severity Scale (EDSS) at 2 years (secondary endpoint).

The exact mechanism of action of FAE has not been established but it is generally thought that effects are mediated through depletion in intracellular glutathione (GSH) stores associated with a switch from an inflammatory Th1 to a more anti-inflammatory Th2 immune response, reduction of peripheral CD4+ and CD8+ T-lymphocytes due to apoptosis, and also nuclear factor kappa B (NF-κB)-dependent down-modulation of inflammatory cytokines and adhesion molecule expression (Mrowietz et al, Trends Mol. Med. 2005 Jan;11(1):43-8). More recently, it was proposed that DMF could also act through induction of type II dendritic cells (Ghoreschi et al., J Exp Med. 2011; 208(11):2291-303). Finally, data also suggests a direct anti-oxidant and neuroprotective effect mediated through nrf2 (Gold et al., Clin Immunol. 2012 Jan; 142(1):44-8).

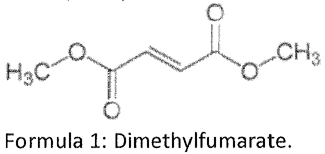

Another drug candidate, teriflunomide (Genzyme) ((Z)-2-cyano-3-hydroxy-but-2-enoic acid-(4'-trifluoromethylphenyl)-amide) (Formula 2) also has an excellent safety profile in Phase III trials where data on more than 2500 patients has already been presented (TEMSO study, O'Connor et al, N Engl J Med. 201;365(14):1293-303, TENERE study Press release, Sanofi, 20 Dec 2012, TOWER study, Press release, Sanofi, 1 Jun 2012). The most important side effects seen in Phase III trials were diarrhea, hair thinning and elevation of transaminases. Teriflunomide's safety is supported through extensive use of its prodrug leflunomide (Arava) in rheumatoid arthritis since its initial approval in 1998. However, clinical efficacy of teriflunomide against MS was only in the range of the conventional agents (indirect comparison to Interferons and direct comparison Glatiramer acetate) and many patients do experience relapses and progression of disability. In fact, for the 7 mg dose, the TENERE study found a higher relapse rate compared with Interferon and the TOWER study even found no significant difference in 12-week sustained accumulation of disability compared with placebo.

More specifically, TEMSO, a randomized, double-blind, placebo-controlled Phase III trial of teriflunomide 7 and 14 mg po once-daily in 1088 RRMS patients showed that teriflunomide 7 and 14 mg significantly reduced annualized relapse rate (ARR) by 31.2% and 31.5% at 2 years compared to placebo (primary endpoint). The risk of disability progression was reduced by 24 and 30% for teriflunomide 7 and 14 mg, respectively. Teriflunomide also reduced the brain disease activity on a range of magnetic resonance imaging measures including reduction of the burden of disease by 39 and 67% for teriflunomide 7 and 14 mg, respectively, compared to placebo. Teriflunomide 7 and 14 mg doses were well tolerated, with treatment emergent adverse events including diarrhoea, nausea and alanine transferase increases were reported in similar number of patients. No serious opportunistic infections occurred in patients treated with teriflunomide. Further results showed that teriflunomide 7 and 14mg significantly increased the time to first relapse by 53.7 and 56.5% during the two years of the study compared to 45.6% on placebo, respectively (TEMSO study, O'Connor et al, N Engl J Med. 201;365(14):1293-303 and Press release, Sanofi-Aventis, 30 Aug 2010 and Press release, Sanofi, 5 Oct 2011).

On the other hand, TENERE, a randomized, open-label Phase III trial in 324 patients with RRMS to assess the effectiveness of 2 doses of teriflunomide 7 and 14 mg po tablet once-daily vs interferon-β1a showed no statistical superiority between the Rebif and teriflunomide arms (7 mg and 14 mg) on risk of treatment failure, which was defined as the occurrence of a confirmed relapse or permanent treatment discontinuation for any cause, whichever came first. However, the teriflunomide 7 mg dose showed a higher relapse rate (0.410) than the 14 mg daily dose (0.259) and Rebif (0.216). Most adverse events observed in the teriflunomide arms were mild in severity, including nasopharyngitis, diarrhea, hair thinning, and back pain. These occurred with a higher incidence than in the Rebif arm. The most common adverse events observed in the Rebif arm were increases in alanine aminotransferase levels, headache and flu-like symptoms. These occurred with a higher incidence than in the teriflunomide arms. There were no deaths in the trial (Press release, Sanofi, 20 Dec 2012).

TOWER, a multi-center, randomized, double-blind, placebo-controlled Phase III trial in 1169 RRMS patients, to evaluate 2 doses of teriflunomide 7 and 14 mg po tablet once-daily versus placebo showed that patients receiving teriflunomide 14 mg had a significant reduction of 36.3% in annualized relapse rate and 31.5% reduction in the risk of 12wk sustained accumulation of disability compared to placebo. In the 7 mg group a significant reduction in annualized relapse rate was observed compared to placebo but there was no significant difference observed for the risk of 12wk sustained accumulation of disability. The most common types of adverse events reported more frequently in the teriflunomide arms were headache, ALT elevations, hair thinning, diarrhoea, nausea and neutropenia (Press release, Sanofi, 1 Jun 2012). Thus, the clinical usefulness of a 7 mg po once-daily teriflunomide dose has not been convincingly established.

Another Phase III study, TOPIC, is underway in early MS or clinically isolated syndrome. Teriflunomide is also being evaluated together with interferon-ß in the Phase III TERACLES trial. With up to 10 years of continuous use in a Phase II extension, teriflunomide has the longest clinical experience of any investigational oral MS therapy.

Teriflunomide was also used in a Phase II combination trial as add-on therapy to IFN where a significant effect on MRI endpoints was observed for 7 and 14 mg doses while no significant effect was seen for the reduction in annualized relapse rate (Freedman, Neurology. 2012 Jun 5;78(23):1877-1885). In another Phase II combination trial Teriflunomide at 7 mg or 14 mg added to glatiramer acetate was more effective than placebo added to in reducing T1-Gd lesions (Freedman et al. Neurology. 2010;74(9):A293.).

Teriflunomide selectively and reversibly inhibits dihydro-orotate dehydrogenase (DHODH), a mitochondrial enzyme required for de novo pyrimidine synthesis. De novo pyrimidine synthesis is required for fast proliferating cells such as activated lymphocytes to meet their needs in DNA, lipid, and sugar metabolism. These effects finally result in strong anti-inflammatory properties through reduced activation and expansion of T- and B-cells in response to autoantigens without apparent cytotoxicity. Teriflunomide has also demonstrated efficiency in inhibiting T-cell-dependent antibody production, suggesting that it modulates the interaction between T cells and B cells. Other effects include reduction of migratory capability of T cells, a diminished ability for exposed T cells to activate monocytes, induction of naïve T cells to favor anti-inflammatory Th-2 differentiation. Cells that rely on DHODH-independent salvage pathways for pyrimidine synthesis (e.g. cells of the hematopoietic system and the gastrointestinal lining) are largely unaffected by teriflunomide's antiproliferative effects.

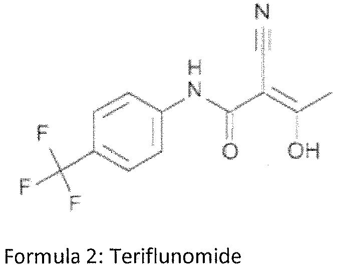

Notwithstanding the above reported works and (partial) progresses, it is still the case that all available agents are only partly effective in halting ongoing inflammatory tissue damage and clinical progression of MS. The reason why therapies are only moderately effective may be seen in the complex and heterogeneous MS pathogenesis where targeting only one aspect of the disease may not suffice to completely stop the disease process. One strategy with the potential to increase treatment efficacy is to combine two or more drugs with distinct modes of action. Such combinations have, for example, been generally described in WO 2007/006307 (salts of fumaric acid monoalkylesters with a multitude of other drugs, see pages 20-25) and specifically claimed in WO 2011/100589 (fumaric acid esters such as dimethylfumarate with either glatiramer acetate or interferon beta). The above described experimental combination therapies with teriflunomide are further examples, but they have not resulted in unequivocally positive results.

Thus, while major advances in MS therapy have already been made, there is still a large unmet need for drugs with improved effectiveness, less side effects, better tolerability and more convenience. The present invention to treat MS with a fixed combination of teriflunomide and dimethylfumarate attempts to address these needs.

### SUMMARY OF THE INVENTION

This invention is in its broadest aspect directed toward novel combination of oral agents to treat multiple sclerosis, i.e. a combination of teriflunomide and dimethylfumarate. These two active ingredients may be present in any pharmaceutically acceptable administration form of either of them. Such pharmaceutically acceptable administration forms, as used herein, include any pharmaceutically acceptable and therapeutically effective crystalline and non-crystalline forms, solvates or hydrates, and in the case of teriflunomide its Z- and E-enolic forms and mixtures thereof and also its prodrug leflunomide. A further component of the claimed oral pharmaceutical composition according to the present invention is one or more pharmaceutically acceptable excipients. The term "excipient" as used in this application is to be understood broadly and encompasses any pharmaceutically acceptable inactive substance that may be present in an oral pharmaceutical administration form, including (but not limited to) fillers, diluents, binders, matrix formers, disintegrants, lubricants, sustained release agents, coating agents and the like.

This invention also provides a pharmaceutical composition containing dimethylfumarate and teriflunomide as the sole active ingredients, together with one or several pharmaceutically acceptable excipients, which is suitable for once daily administration.

One preferred embodiment of the invention provides for administering a novel fixed-dose combination for once daily oral use of a first active component which is dimethylfumarate, at a dose that is therapeutically effective when used alone, and of a second active component which is teriflunomide, at a dose that has not shown therapeutic efficacy when used alone. Therefore, according to a preferred aspect of the invention, this pharmaceutical composition contains dimethylfumarate at a dose range of 500 mg to 750 mg and teriflunomide at a dose range of 1 mg to 6 mg.

According to the present invention, the combination products described herein will show better efficacy (as measured by reduction of annualized relapse rate and/or progression of disability) than dimethylfumarate alone. In addition, the inventive combination products will not show an increase in severe side effects compared with dimethylfumarate alone.

A further embodiment of the invention provides for administering a novel fixed-dose combination for once daily oral use of a first component which is dimethylfumarate, in a daily dose below the daily doses shown to be therapeutically effective for MS in DEFINE and CONFIRM studies, and of a second active component which is teriflunomide at a dose that has not shown therapeutic efficacy when used alone. The combination product according to this preferred aspect of the invention will show a non-inferior efficacy (as measured by reduction of annualized relapse rate and/or progression of disability) compared with each dimethylfumarate and teriflunomide, when used at therapeutically effective doses alone. Thus, in a preferred aspect of the present invention the composition contains dimethylfumarate at a dose range of 125 mg to 500 mg and teriflunomide at a dose range of 1 mg to 6 mg.

A further embodiment of the invention provides for administering a novel fixed-dose combination for twice daily oral use of a first active component which is dimethylfumarate, at a dose that is therapeutically effective when used alone, and of a second active component which is teriflunomide, at an absolute daily dose that has not shown therapeutic efficacy when used alone. According to the invention, the combination product will show a significantly better efficacy (as measured by reduction of annualized relapse rate and/or progression of disability) then dimethylfumarate alone. In addition, this combination will not show a statistically significant increase in fatal events and/ or severe adverse events compared with dimethylfumarate alone.

A further embodiment of the invention provides for administering a novel fixed-dose combination for twice daily oral use of a first component which is dimethylfumarate, in a dose below the doses shown to be therapeutically effective in DEFINE and CONFIRM studies, and of a second active component which is teriflunomide at an absolute daily dose that has not shown therapeutic efficacy when used alone. This combination product will show a non-inferior efficacy (as measured by reduction of annualized relapse rate and/or progression of disability) compared with each dimethylfumarate and teriflunomide, when used at therapeutically effective doses alone.

In further embodiments of the invention, teriflunomide is replaced in all of the above combinations by its prodrug leflunomide at a bioequivalent dose (as measured by teriflunomide pharmacokinetics).

### DETAILED DESCRIPTION OF THE INVENTION

This invention is related to a method of treating MS in a human patient in need of such treatment and comprises administering to said patient a combination therapy in a single oral dosage form (e.g. a tablet or capsule) of dimethylfumarate and teriflunomide (or its prodrug leflunomide) which is more effective than the single agents alone and/or has reduced side effects and better tolerability than the single agents alone and/or can be given in a reduced frequency.

Although dimethylfumarate and teriflunomide have each been used individually for the treatment of MS, the agents have not been used in combination for the treatment of MS. The inventors have recognized that the added or synergistic effect of these two active ingredients will most likely be due to the fact that both agents have different molecular targets and many non-overlapping modes of action in the pathophysiology of MS. Mainly, dimethylfumarate has significant neuroprotective properties in addition to its leading immunomodulatory effects, but no immunosuppressive effects. Teriflunomide on the other hand has a broad inhibitory effect on rapidly proliferating cells including B cells, T cells and cells of the innate immune system, and thus, an immunosuppressive activity. According to the present invention, the specific spectrum of activities of these respective agents allows for a particularly advantageous efficacy and side effect profile of the combination.

In a preferred combination therapy according to the present invention a drug such as teriflunomide that does not have a significant clinical effect when used alone still has a significant additional effect when used in combination with another drug (dimethylfumarate) that is effective alone. In addition, the combination will be associated with a similar or even more benign side effect profile compared to the single drugs while efficacy is retained. Finally, combining a drug usually used in a twice daily regime such as dimethylfumarate with a drug usually used in a once daily regime such as teriflunomide may allow creating a once daily combination drug with non-inferior efficacy to both drugs when used alone and, depending on specific side effect profile and dose, no increase in side effects.

One preferred composition according to the present invention is intended for once daily use and consists of component 1) Dimethylfumarate at a dose range of 500 mg to 750 mg and of component 2) Teriflunomide at a dose range of 1 mg to 6 mg and of components 3) (excipients) which are required for the pharmaceutical formulation. A particularly preferred combination according to this aspect of the invention will contain 625 mg dimethylfumarate and 5 mg teriflunomide. Further preferred combinations contain 500 mg dimethylfumarate and 6 mg teriflunomide, 500 mg dimethylfumarate and 5 mg teriflunomide, 500 mg dimethylfumarate and 4 mg teriflunomide, 500 mg dimethylfumarate and 3 mg teriflunomide, 625 mg dimethylfumarate and 4 mg teriflunomide, 625 mg dimethylfumarate and 3 mg teriflunomide, 625 mg dimethylfumarate and 2 mg teriflunomide, and 625 mg dimethylfumarate and 1 mg teriflunomide. Further preferred embodiments contain 750 mg dimethylfumarate in combination with 1, 2, 3, 4 or 5 mg teriflunomide.

Another preferred composition according to the invention is intended for once daily use and consists of component 1) Dimethylfumarate at a dose range of 125 mg to 500 mg and of component 2) Teriflunomide at a dose range of 1 mg to 6 mg and of components 3) (excipients) which are required for the pharmaceutical formulation. Preferably, the composition according to this aspect of the invention is intended for once daily use and consists of component 1) Dimethylfumarate at a dose range of 125 mg to 375 mg and of component 2) Teriflunomide at a dose range of 1 mg to 6 mg and of components 3) (excipients) which are required for the pharmaceutical formulation. Particularly preferred combinations according to this aspect of the invention contain 375 mg dimethylfumarate in combination with 2, 3, 4, 5 or 6 mg teriflunomide, or 375 mg dimethylfumarate in combination with 1, 2, 3, 4 or 5 mg teriflunomide, or 375 mg dimethylfumarate in combination with 5 mg teriflunomide, or 250 mg dimethylfumarate in combination with 2, 3, 4, 5 or 6 mg teriflunomide, or 125 mg dimethylfumarate in combination with 3, 4, 5 or 6 mg teriflunomide.

A third preferred composition is intended for twice daily use and consists of component 1) Dimethylfumarate at a dose range of 250 mg twice daily to 375 mg twice daily and of component 2) Teriflunomide at a dose range of 0.5 mg twice daily to 3 mg twice daily and of components 3) which are required for the pharmaceutical formulation. A particularly preferred combination according to this aspect of the invention will contain 375 mg dimethylfumarate and 2.5 mg teriflunomide. Further preferred combinations contain 250 mg dimethylfumarate and 3 mg teriflunomide, 250 mg dimethylfumarate and 2.5 mg teriflunomide, 250 mg dimethylfumarate and 2 mg teriflunomide, 250 mg dimethylfumarate and 1.5 mg teriflunomide, 375 mg dimethylfumarate and 2 mg teriflunomide, 375 mg dimethylfumarate and 1.5 mg teriflunomide, 250 mg dimethylfumarate and 1 mg teriflunomide, and 375 mg dimethylfumarate and 0.5 mg teriflunomide. Further preferred embodiments contain 375 mg dimethylfumarate in combination with 0.5, 1, 1.5, 2, or 2.5 mg teriflunomide.

A fourth composition is intended for twice daily use and consists of component 1) Dimethylfumarate at a dose range of 60 mg twice daily to 250 mg twice daily and of component 2) Teriflunomide at a dose range of 0.5 mg twice daily to 3 mg twice daily and of components 3) which are required for the pharmaceutical formulation. A particularly preferred combination according to this aspect of the invention will contain 150 mg dimethylfumarate and 2.5 mg teriflunomide. Further preferred combinations contain 125 mg dimethylfumarate and 5 mg teriflunomide, 150 mg dimethylfumarate and 3 mg teriflunomide, 125 mg dimethylfumarate and 2.5 mg teriflunomide, 125 mg dimethylfumarate and 2 mg teriflunomide, 125 mg dimethylfumarate and 1.5 mg teriflunomide, 150 mg dimethylfumarate and 2 mg teriflunomide, 150 mg dimethylfumarate and 1.5 mg teriflunomide, 125 mg dimethylfumarate and 1 mg teriflunomide, and 150 mg dimethylfumarate and 0.5 mg teriflunomide. Further preferred embodiments contain 180 mg dimethylfumarate in combination with 0.5, 1, 1.5, 2, or 2.5 mg teriflunomide.

Compositions according to the invention that are intended for once or twice daily use according to the present invention include those wherein Teriflunomide is present in the form of bioequivalent doses (as measured by teriflunomide pharmacokinetics) of its prodrug Leflunomide. Teriflunomide forms from leflunomide via rearrangement and ring opening.

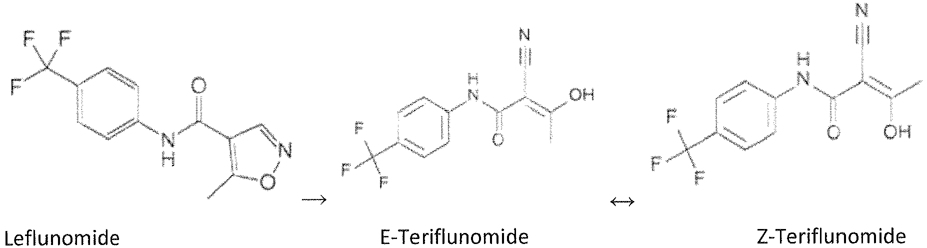

According to preferred aspects of the present invention, teriflunomide is used at doses that are below demonstrated therapeutic effectiveness when used alone. Thus, the therapeutic benefit of the preferred inventive combinations is caused by unexpected additional effects provided by teriflunomide at doses heretofore thought to be probably ineffective. Dimethylfumarate has been demonstrated to induce a disease modifying and disease intervening effect as measured on annual relapse rates and progression of disability in patients. Seen in combination with its different (orthogonal) mechanism of action than teriflunomide and in the context of the demonstrated safety, with side effects primarily consisting of mild tolerability issues such as diarrhea, nausea, stomach pain, dimethylfumarate is an ideal partner for combination with teriflunomide. Most pharmaceutical drugs have S-shaped dose-response curves or bell shaped dose response curves. The addition of another pharmacological agent such as teriflunomide would thus be expected to shift the aggregated dose-response curve in a favorable manner and thus be of major clinical and therapeutic utility.

A further aspect of the present invention is a suitable pharmaceutical formulation or once or twice daily oral administration of the inventive combination of dimethylfumarate and teriflunomide. The formulation can be any oral formulation, but is preferably a tablet or pellet formulation, or a capsule formulation, e.g. a gelatin capsule.

According to a particular aspect of this invention, the two active ingredients are present in different portions of the oral formulation that are designed to release the respective active ingredient with different speeds.

Thus, according to this aspect, the invention also provides a pharmaceutical composition for oral use against MS that contains dimethylfumarate and teriflunomide as the active ingredients, wherein the dimethylfumarate is contained in a portion of the composition that provides for prolonged release of the active ingredient and the teriflunomide is contained in a portion of the composition that provides for rapid release of the active ingredient.

In a particular embodiment of the invention the dimethylfumarate is contained in a prolonged release matrix portion of a tablet and the teriflunomide is contained in a coating surrounding the matrix portion. In a particular embodiment, the teriflunomide is contained in an outer enteric coating surrounding the matrix portion of the tablet, which embeds and surrounds the dimethylfumarate. In an alternative embodiment teriflunomide is contained in a separate water-soluble or readily water-disintegratable layer between the core and the outer enteric coating, or as the outermost layer. Suitable tablets according to the invention may contain lactose (e.g. tablettose) or microcrystalline cellulose as a filler, hydroxypropylcellulose or hydroxypropylmethylcellulose as matrix-forming retarding agent and magnesium stearate as a lubricant, and they may be coated, e.g. with a film coat or an enteric coat or a drug-containing layer. Useful coating agents include acrylic polymers, e.g. from the Eudragit series, such as Eudragit L30D.

Prolonged or sustained release matrix formulations that are suitable to serve as prolonged release matrix portions of the inventive tablets are disclosed in WO 2010/079222 the disclosure of which is incorporated herein in its entirety. Such prolonged release matrix formulations may be provided with an additional fast release coating containing teriflunomide. Alternatively, teriflunomide may be added to an enteric coating as provided in many of the examples of WO 2010/079222.

Thus, a coated erosion matrix tablet can be used to formulate the combination of dimethylfumarate and teriflunomide according to the present invention. Alternatively, the two active ingredients can also be put into respective controlled release (CR) and immediate release (IR) microtablets or pellets, which can then be filled into gelatin capsules or sachets. In such an embodiment dimethylfumarate will again be in a CR microtablet or pellet, whereas teriflunomide will be in the IR microtablet or pellet.

### EXAMPLES

The following examples are offered to illustrate various aspects of the invention and are not to be construed as to limit the invention in any way.

### Example 1. Clinical trial design to demonstrated the proposed synergistic effects.

A clinical trial will include multiple sclerosis patients of Remitting-Relapsing type diagnosed on McDonald criteria, with a baseline Expanded Disability Status Scale (EDDS) between 0 and 5 and either at least one relapse within the last 12 months of randomisation and a previous MRI scanning showing lesions consistent with multiple sclerosis or GdE lesions on MRI scan done within 6 months of randomisation. Excluded will be patients with a relapse within 50 days of randomisation or no stabilization from a previous relapse. Patients who within the last year have been treated with T-cell or T-receptor vaccination, total lymphoid irradiation or therapeutic monoclonal antibody treatment, who had been treated with mitoxantron or cyclophosphamide within the last year of randomisation were also excluded. Also patients who within 6 months of randomisation had been treated with cyclosporin, azathioprin, methotrexate or plasmapheresis were excluded. Patients with previous gastrointestinal disease such as ulcus duodeni, gastritis or pancreatic disease will be excluded. Patents with lymphocytopenia, low white blood cell count or calculated creatinine clearance of < 60 mL/min at baseline will also be excluded.

The trial will be approved by all relevant Competent Agencies as well as all relevant Ethic Committees. The trial will be a randomized, double blind, double-dummy, placebo controlled parallel group design testing 3 active treatment arms and a placebo arm:
1: a combination tablet consisting of 500mg prolonged release DMF and the instant release 6 mg teriflunomide in a single formulated enteric coated tablet;
2: a teriflunomide 6 mg plus placebo DMF enteric coated tablet;
3: a 500mg DMF dose with a teriflunomide placebo enteric coated tablet;
4: a placebo DMF and placebo teriflunomide enteric coated tablet.

The placebo will also document the sensitivity of all 3 active arms.

Primary endpoints will be based on MRI scans using the number and volume of new GdE lesions on post contrast T1-weighed sequences as well as the number of T2-weighed enlarged lesions. Secondary endpoints will be the number of relapses monitored monthly and the EDDS that will be assessed at 12 weeks interval from baseline as well as brain atrophy. Safety will be followed closely in particular on differential count of white blood cells, liver enzyme values, gastrointestinal side effects and infections. Laboratory examination will be performed every 4 weeks and general safety as assessed by the reporting of SAE's and AE's and neurological and physical examination. Treatment time will be 24 weeks initially for the evaluation of the primary endpoint followed by a blinded 24 week follow up where the active treatment groups will continue their randomized treatment and the patients on placebo will transferred to active treatment with a continued blinded dosing where they will receive an active combination tablet consisting of 500mg DMF in the prolonged release formulation and the instant release 6mg teriflunomide in an enteric coated tablet. The number of patients will be 400 with a 1:1:1:1 randomisation between the groups based on previously reported mean MRI lesions reduction data with DMF treatment and teriflunomide treatment, assuming a 20% reduction in the number of new GdE lesions, a power of 80% to detect a treatment effect based on a two-sided 5% significance level.

All MRI evaluations will be performed centrally by an experienced neuro-radiologist. An interim analysis is planned after all patients have completed the first 24 weeks comparing each active arm against placebo and furthermore the combination tablet treatment arm compared to each of the single treatment arms. Analysis will be performed using adaptive design and closed analysis with no adjustment of the significance level.

Patients who enter the trial after screening and randomisation will follow a schedule of investigations running at week 2 from randomisation/baseline, at week 4 and then every 4 weeks for the entire trial period with an 8 week follow up for each patient at the end of treatment. Plasma samples for population kinetics will be sampled at baseline, week 4, 8, 12, 24, 36 and 48 of the trial schedule.

Patients who discontinue prematurely will be offered alternative treatment at the discretion of the investigators. An independent Safety Data Monitoring Committee will monitor safety data on a monthly basis, review all SAE's and possible infections and decide on out of schedule laboratory or other safety measures including a for safety reasons premature discontinuation of patients. The trial will be carried out in approximately 50-60 centers in 6-8 countries.

### Example 2. Formulation Example

An enteric coated tablet containing an erosion matrix core with a film coat is used to formulate the combination of dimethylfumarate and teriflunomide according to the present invention.

The enteric coated tablet consists of an erosion matrix core hosting the dimethylfumarate covered by a film coating hosting teriflunomide and an outer thin enteric coating. The enteric coating rapidly dissolves when reaching the small intestine and releases teriflunomide in the duodenum at weakly alkaline pH values. In contrast, due to the erosion matrix the dimethylfumarate is released in a prolonged manner (controlled release) over several hours.

The composition of the tablet core for a 125 mg DMF strength is shown in the following table.

| Ingredient | Amount of ingredient/275 mg core tablet weight [mg] | Amount of ingredient/core tablet weight [wt.-%] |
|---|---|---|
| Dimethylfumarate | 125 | 45.45 |
| Lactose (Tablettose 100) | 135.7 | 49.35 |
| Hydroxypropylcellulose (HPC-SL) | 12 | 4.36 |
| Silica (Aerosil) | 0.3 | 0.11 |
| Magnesium stearate | 2 | 0.73 |

These 275 mg cores are then coated with 5 wt.-% of an aqueous PVA-solution containing e.g. 5 mg teriflunomide so as to obtain a teriflunomide-containing layer that rapidly dissolves when being contacted with water.

A thin enteric coating is then applied to these coated cores. The coating has the following composition:

| | |
|---|---|
| Eudragit L30D55* | 7.56 mg (2.75%**) |
| Triethylcitrate | 0.76 mg |
| Cutina GMS V | 0.23 mg |
| Tween 80 | 0.09 mg |

| | |
|---|---|
| * solid contents are listed (Eudragit is a suspension with 30% solids), ** theoretically applied coating composition, the actually applied coat is approximately 2 wt.-%. | |

Further tablet strengths with varying combinations of a tablet core with prolonged release formulation of DMF containing 60 mg, 150 mg, 250 mg, 375 mg and 500 mg combined with 1 mg, 1.5 mg, 2 mg, 2.5 mg, 3 mg, 4 mg, 5 mg and 6 mg of teriflunomide embedded in the film coating will apply.

The manufacture and coating steps are carried out by known methods such as, e.g., described in WO 2010/079222, examples 21 and 22.

## Claims

1. A pharmaceutical composition suitable for the oral treatment of multiple sclerosis consisting of dimethylfumarate and teriflunomide as active ingredients and one or more pharmaceutically acceptable excipients.

2. Pharmaceutical composition according to claim 1, which is suitable for once daily administration.

3. Pharmaceutical composition according to claim 2 containing dimethylfumarate at a dose range of 500 mg to 750 mg and teriflunomide at a dose range of 1 mg to 6 mg.

4. Pharmaceutical composition according to claim 3 containing dimethylfumarate at a dose range of 625 mg and teriflunomide at a dose range of 5 mg.

5. Pharmaceutical composition according to claim 2 containing dimethylfumarate at a dose range of 125 mg to 500 mg and teriflunomide at a dose range of 1 mg to 6 mg.

6. Pharmaceutical composition according to claim 5 containing dimethylfumarate at a dose range of 375 mg and teriflunomide at a dose range of 5 mg.

7. Pharmaceutical composition according to claim 1, which is suitable for twice daily administration.

8. Pharmaceutical composition according to claim 7 containing dimethylfumarate at a dose range of 250 mg to 375 mg and teriflunomide at a dose range of 0.5 mg to 3 mg.

9. Pharmaceutical composition according to claim 7 containing dimethylfumarate at a dose range of 375 mg and teriflunomide at a dose range of 2 mg.

10. Pharmaceutical composition according to claim 7 containing dimethylfumarate at a dose range of 60 mg to 250 mg and teriflunomide at a dose range of 0.5 mg to 3 mg.

11. Pharmaceutical composition according to claim 7 containing dimethylfumarate at a dose range of 125 mg and teriflunomide at a dose range of 5 mg.

12. Pharmaceutical composition according to any of the preceding claims, wherein the dimethylfumarate is contained in a portion of the composition that provides for prolonged release of the active ingredient and the teriflunomide is contained in a portion of the composition that provides for rapid release of the active ingredient.

13. Pharmaceutical composition according to claim 12, wherein the dimethylfumarate is contained in a prolonged release matrix portion of a tablet and the teriflunomide is contained in a coating surrounding the matrix portion.

14. Pharmaceutical composition according to claim 13, wherein the teriflunomide is contained in an outer enteric coating surrounding the matrix portion of the tablet.

15. A method of treating multiple sclerosis (MS) in a human patient in need of such treatment which comprises administering to said patient a pharmaceutical composition for oral use that contains dimethylfumarate and teriflunomide (or its prodrug leflunomide).
